# EUROPÄISCHE PATENTSCHRIFT

(11) **EP 0 969 898 B1**
(45) Veröffentlichungstag und Bekanntmachung des Hinweises auf die Patenterteilung: **09.07.2003**
(21) Anmeldenummer: 98905417.6
(22) Anmeldetag: 10.02.1998
(51) Int. Cl.: A61N 1/14

(54) **VORRICHTUNG ZUR HEMMUNG VON SCHMERZIMPULSEN IN DEN NERVENLEITUNGEN VON MENSCH UND TIER**
DEVICE FOR INHIBITING PAIN IMPULSES IN THE NERVE PATHWAYS OF HUMAN BEINGS AND ANIMALS
DISPOSITIF POUR INHIBER LES IMPULSIONS DOULOUREUSES DANS LES VOIES NERVEUSES DE L'HOMME ET DE L'ANIMAL

(30) Priorität: 25.03.1997 DE 19712455
(43) Veröffentlichungstag der Anmeldung: 12.01.2000
(73) Patentinhaber: Festl, Monika, 90453 Nürnberg (DE)
(72) Erfinder: DEINLEIN-KALB, Hans, D-90453 Nürnberg (DE); DEINLEIN-KALB, Marianne, D-90453 Nürnberg (DE)
(74) Vertreter: Brose, Manfred, Dr.
(86) Internationale Anmeldenummer: EP9800732
(87) Internationale Veröffentlichungsnummer: WO98042404

(56) Entgegenhaltungen:
- EP-A- 0 296 248
- WO-A-92/10113
- DE-A- 3 500 379
- GB-A- 2 025 237
- US-A- 5 607 453

## Beschreibung

Die Erfindung betrifft eine Vorrichtung zur Hemmung von Schmerzimpulsen in den Nervenleitungen von Mensch und Tier.

Schmerz ist eines der häufigsten Symptome einer Krankheit oder einer Gewebeschädigung oder einer Störung des Gewebestoffwechsels. Der Schmerz wird dann spürbar, wenn mechanische, thermische, chemische oder elektrische Reize einen gewissen Schwellenwert überschreiten.

In der Schmerzbekämpfung werden therapeutisch zum Teil sehr stark wirkende Analgetika eingesetzt mit ebenso starken Nebenwirkungen für Mensch, Tier und Umwelt.

Die Schmerzleitung in den Nervenbahnen von Mensch und Tier erfolgt durch Ionen, die an der elektrisch geladenen Membran der Nervenleitung entlangwandern. Eine ruhende Membran ist außen positiv und innen negativ geladen. Das Potential oder die Spannung der Außenseite gegen die Innenseite beträgt im schmerzfreien Ruhezustand (Ruhepotential) etwa 70 Millivolt (mV). Im Augenblick der Erregung durch Schmerzen kehren sich die Ladungen um, wodurch beim Menschen das Potential während 1/1000 sec um etwa 100 mV nach der negativen Seite, d.h. bis zu einem Wert von - 30 mV von der Außen- gegen die Innenseite abfällt. Die Frequenz der durch die lonenwanderung entstehenden an- und absteigenden Phase des Spitzenpotentials ist etwa der zu übertragenden Schmerzintensität proportional. Die Leitungsgeschwindigkeit der Nervenaktion kann beim Menschen bis zu 100 m/sec. betragen.

Aus DE 1 701 259 U (Schotten) ist eine Vorrichtung zum Ausgleich von Nervenstörungen bekannt. Die Vorrichtung besteht aus einem elektrischen Kondensator in einem Gehäuse mit zwei Außenkontakten, die mit den beiden Anschlußleitungen des Kondensators verbunden sind. Dieser bekannten Vorrichtung haftet der Nachteil an, daß die Kapazität des elektrischen Kondensators auf einen Bereich von 0,02 bis höchstens 2,00 Mikrofarad begrenzt ist. Die Erfahrung hat jedoch gezeigt, daß die Vorrichtung durch diese Begrenzung der Kapazität wirkungslos ist.

Der Erfindung liegt daher die Aufgabe zugrunde, die Nachteile der bekannten Vorrichtung in Bezug auf ihre Wirksamkeit zu beseitigen.

Diese Aufgabe wird nach der Erfindung dadurch gelöst, daß die gattungsgemäße Vorrichtung auch nach dem Kennzeichen des Anspruchs 1 ausgelegt ist.

In den Unteransprüchen werden Ausgestalltungen und Fortbildungen der Erfindung beansprucht.

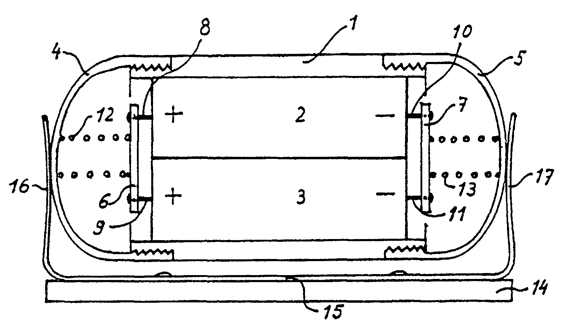

Nachfolgend wird der Erfindungsgegenstand beschrieben. In der Zeichnung ist
ein Ausführungsbeispiel des Erfindungsgegenstandes für eine manuelle Anwendung
wiedergegeben.

Der Erfindungsgegenstand besteht aus einem Rohrgehäuse 1, den Elektrokondensatoren 2, 3, den Metallschalen 4, 5, den Kontaktplatten 6, 7, den Leitungsdrähten 8, 9, 10, 11, den Druckfedern 12, 13 sowie der Federhaltung 14 mit den Federbügeln 16, 17 und dem Verbindungssteg 15.

Im Rohrgehäuse 1, das aus einem nichtleitenden Material besteht, sind parallel zur Mittelachse des Rohrgehäuses 1 beispielweise zwei Stabkondensatoren 2, 3 eingebaut, aus deren zylindrischen Enden die Leitungsdrähte 8, 9, 10 und 11 herausragen. Die jeweils gleichpoligen Enden der Leitungsdrähte 8, 9 und 10, 11 sind mit den Kontaktplatten 6, 7 verlötet, so daß sich eine Parallelschaltung der Kondensatoren 2, 3 ergibt. Beidseitig sind in die jeweilige Rohröffnung des Rohrgehäuses 1 Metallschalen 4, 5 eingeschraubt, die über Druckfedern 12, 13, den Kontaktplatten 6, 7 und den Leitungsdrähten 8, 9, 10, 11 einen ständigen Kontakt zwischen den Metallschalen 4, 5 und den Kondensatoren 2, 3 herstellen. Die Anzahl der Kondensatoren richtet sich nach der konstruktiven Gestaltung des Erfindungsgegenstandes sowie nach der Größe der gewünschten Kondensatorkapazität.

In Bereitschaftsstellung, d.h. wenn der Erfindungsgegenstand nicht im Gebrauch ist, wird dieser in einer Federhalterung 14 dergestalt gehaltert, daß der Erfindungsgegenstand zwischen zwei leitenden Federbügeln 16, 17 eingeklemmt wird wodurch zugleich über den Federbügel 16, den Verbindungssteg 15 und den Federbügel 17 eine Durchflutungsverbindung zwischen der Metallschale 4 und der Metallschale 5 hergestellt wird. Durch diesen Kurzschluß der Kondensatoren 2, 3 wird sichergestellt, daß der Erfindungsgegenstand immer potential- bzw. ladungsfrei zum Einsatz kommt, wenn dieser aus der Federhalterung herausgenommen wird.

Beim Einsatz des Erfindungsgegenstandes gegen eine Schmerzattacke wird das Rohrgehäuse 1 mit den Fingern derart umklammert, daß die Metallschale 5 (Minuspolseite) gegen die hohle Innenhand zur Anlage kommt. Das gegenüberliegende Ende mit der Metallschale 4 (Pluspolseite) wird mit leichtem Druck gegen die Schmerzstelle des Körpers gedrückt. Damit wird zwischen der schmerzsignalführenden Nervenleitung durch Gewebe und Hautoberfläche hindurch mit der Pluspolseite der Kondensatoren 2, 3 eine leitende Verbindung hergestellt. Die bei der Spannungsumkehr entstehenden pulsartigen Spannungsspitzen in der Nervenleitung werden so durch den Verschiebefluß mit den Kondensatoren 2, 3 verkleinert oder vollständig geglättet. Damit signalisiert die betreffende Nervenleitung dem Gehirn Schmerzfreiheit.

Versuche haben gezeigt, daß z.B. bei starken Rücken-, Muskel-, Gelenk- und Ischiasschmerzen sowie bei schmerzhaften Wadenkrämpfen durch den Einsatz des Erfindungsgegenstandes nach etwa vier (4) Minuten die Schmerzintensität schlagartig bzw. schalterartig auf zu Null herabgesetzt wurde. Das bedeutet: Je stärker der Schmerz und damit analog die Spannungs- und Impulsfrequenz in der Nervenleitung ist, desto höher und wirksamer ist die Kompensation durch die Kondensatoren 2, 3.

Etwas länger, etwa zehn (10) Minuten, muß die Anwendung ausgedehnt werden, wenn die Nervenleitung tieferliegender Organe behandelt werden soll, weil mögliche Luftzwischenräume oder Knochenteile den Verschiebefluß zu den Kondensatoren 2, 3 abschwächen.

Die Zeit für die anschließende vom Patienten subjektiv beurteilte Schmerzfreiheit nach einer Behandlung wurde mit etwa zwei (2) Stunden bis zu mehreren Tagen angegeben, wobei starke Schmerzen zeitlich eher länger kompensiert bleiben als weniger starke Schmerzen.

Es versteht sich, daß mit dem Erfindungsgegenstand auch Spannungsspitzen in den sogenannten motorischen Nervenleitungen, z.B. bei einem Tremorbefall, verkleinert oder geglättet werden können.

Es wurde auch gefunden, daß eine Kurzschlußbrücke zwischen den Plus- und den Minuspolen der Kondensatoren 2, 3 die dynamische Kapazität der Kondensatoren 2, 3 vergrößert. Dieses hat den Vorteil, daß die Kondensatoren 2, 3 statisch permanent nicht mehr aufladbar sind und damit zugleich unerwünschte Entladungsströme und Entladungsspannungen unmöglich gemacht werden.

Der dynamische pulsartige Verschiebefluß um das statische Nullpotential der Kondensatoren 2, 3 gegenüber der Nervenleitung im Bereich von etwa 3 bis 500 Hertz wird dadurch verbessert. Die Federhalterung 15, 16, 17 für die zeitweise Entladung der Kondensatoren 2, 3 kann entfallen.

Kann die Schmerzstelle bzw. die schmerzübertragende Nervenleitung nicht eindeutig lokalisiert werden, dann ist es zweckmäßig, die Schmerzstelle an der Hautoberfläche mit einer Metallfolie, z.B. einer Aluminiumfolie, abzudecken, bei den Extremitäten, z.B. Arm oder Bein, mit der Metallfolie zu umwickeln und die Folien direkt oder mittels Kabel mit den kurzgeschlossenen Kondensatoren 2, 3 zu verbinden. Diese Vorgehensweise ist auch bei Kopfschmerzen unbekannter Genese angezeigt.

Eine weitere sehr effektive Methode der Schmerzbehandlung wird auch durch eine direkte oder indirekte Kontaktierung des Erfindungsgegenstandes mit großvolumigen Arterien erzielt. Wird z.B. der Kontaktpol des Erfindungsgegenstandes gegen die Hautoberfläche direkt an Halsschlagader (Carotis) zur Anlage gebracht, dann wird hierdurch eine Leitungsverbindung zwischen den kurzgeschlossenen Kondensatoren 2, 3 im Erfindungsgegenstand und dem leitfähigen, pulsierenden Blut in der Arterie und damit zugleich ein elektrischer Verschiebefluß zwischen schmerzerregten Nervenleitungen allerorts im Körper des Patienten und dem Erfindungsgegenstand hergestellt. Auch können beide Halsschlagadern gleichzeitig durch eine äußere überbrückung, z.B. mittels einer Metallfolie oder einem leitfähigen Metallhalsband mit dem Erfindungsgegenstand in Kontaktverbindung gebracht werden.

Dieser Vorgang ist so zu verstehen, daß das kapazitive Feld der Kondensatoren 2, 3 im Erfindungsgegenstand auch auf das gesamte Blutvolumen des Patienten ausgedehnt wird. Damit kann auch, außer einer Schmerzblockierung, das im vegetatieven Nervensystem durch die Aufnahme von sogenannten "Elektrosmog" aus der Umwelt oder durch Wetterwechsel angereicherte elektrische Potential abgebaut und so dem Patienten ein besseres Wohlbefinden vermittelt werden.

Es versteht sich, daß der Erfindungsgegenstand, z.B. in Minibauart, auch als Dauerblocker unterhalb der Hautoberfläche in direkter oder indirekter Anlage eine Arterie implantiert werden kann.

Es ist auch möglich, daß sich der Mensch oder das Tier zum Zwecke einer Schmerztherapie in einem Wasserbad befindet, das seinerseits mit kurzgeschlossenen Kondensatoren erhöhter Kapazität verbunden ist.

Der Erfindungsgegenstand ist inzwischen vielfach erfolgreich erprobt worden, wobei die Anwendungserfolgsquote bei intakten, ungeschädigten Nervenleitungen bei nahezu 100 % liegt.

### Bezugszeichenliste

- 1: Rohrgehäuse
- 2: Elektrokondensator
- 3: Elektrokondensator
- 4: Metallschale
- 5: Metallschale
- 6: Kontaktplatte
- 7: Kontaktplatte
- 8: Leitungsdraht
- 9: Leitungsdraht
- 10: Leitungsdraht
- 11: Leitungsdraht
- 12: Druckfeder
- 13: Druckfeder
- 14: Federhalterung
- 15: Verbindungssteg
- 16: Federbügel
- 17: Federbügel

## Patentansprüche

1. Vorrichtung zur Hemmung von Schmerzimpulsen in den Nervenleitungen von Mensch und Tier durch Kontaktierung eines elektrischen Kondensators (2) mit der Hautoberfläche, wobei der Kondensator (2) oder mehrere parallelgeschaltete Kondensatoren (2, 3) in einem Gehäuse (1) mit zwei Außenkontakten (4, 5) untergebracht und die Außenkontakte (4, 5) mit den beiden Anschlußleitungen (8, 10; 9, 11) der Kondensatoren (2, 3) verbunden sind,
**dadurch gekennzeichnet, daß**
der Plus- und der Minuspol der Kondensatoren (2, 3) mittels einer Kurzschlußbrücke unlösbar miteinander verbunden sind und zur Schmerzbehandlung ein Außenkontakt (4, 5) des Kondensators (2, 3) kontaktierend an die Hautoberfläche über einer Schmerzstelle angelegt wird und Spannungsspitzen des Schmerzimpulses durch den Verschiebestrom des Kondensators verkleinert oder vollständig geglättet werden.

2. Vorrichtung nach Anspruch 1,
**gekennzeichnet durch**
großflächige, balliggerundete Metallschalen (4, 5) als Außenkontakte.

3. Vorrichtung nach einem der Ansprüche 1 bis 3,
**dadurch gekennzeichnet, daß**
die Kapazität des Kondensators oder die Gesamtkapazität der parallelgeschalteten Kondensatoren (2, 3) mindestens 10.000 Mikrofarad ( uF) beträgt.

## Claims

1. Device for inhibiting pain pulses in the nerves of humans and animals by contacting an electrical capacitor (2) with the skin surface, wherein the capacitor (2) or several capacitors (2, 3) connected in parallel is or are accommodated in a housing (1) with two external contacts (4, 5) and the external contacts (4, 5) are connected with the two connecting lines (6, 10; 9, 11) of the capacitors (2, 3), **characterised in that** the positive pole and the negative pole of the capacitors (2, 3) are non-detachably connected together by means of a short-circuit bridge and for the treatment of pain an external contact (4, 5) of the capacitor (2, 3) is brought into contact with the skin surface over a place of pain and voltage peaks of the pain pulse are reduced or completely smoothed out by the displaced current of the capacitor.

2. Device according to claim 1, **characterised by** large-area, spherically rounded metal shells (4, 5) as external contacts.

3. Device according to one of claims 1 to 3, **characterised in that** the capacitance of the capacitor or the total capacitance of the capacitors (2, 3) connected in parallel amount to at least 10,000 microfarads (µF).

## Revendications

1. Dispositif pour inhiber des impulsions de douleur dans les circuits nerveux d'hommes et d'animaux par contact d'un condensateur électrique (2) avec l'épiderme, le condensateur (2) ou plusieurs condensateurs (2, 3) montés en parallèle étant logés dans un boîtier (1) avec deux contacts extérieurs (4, 5), et les contacts extérieurs (4, 5) étant reliés aux deux lignes de jonction (8, 10 ; 9, 11) des condensateurs (2, 3),**caractérisé en ce que** le pôle positif et le pôle négatif des condensateurs (2, 3) sont reliés entre eux de façon inamovible au moyen d'un pontage de court-circuit, et un contact extérieur (4, 5) du condensateur (2, 3) est appliqué en contact de l'épiderme sur un point douloureux pour le traitement de la douleur, et les crêtes de tension de l'impulsion de douleur sont réduites ou totalement lissées par le courant de déplacement du condensateur.

2. Dispositif suivant la revendication 1, **caractérisé par** des coques métalliques (4, 5) bombées, de grande surface, comme contacts extérieurs.

3. Dispositif suivant l'une des revendications 1 à 3, **caractérisé en ce que** la capacité du condensateur ou la capacité totale des condensateurs (2, 3) montés en parallèle atteint au moins 10.000 microfarads (µF).
